# EUROPEAN PATENT SPECIFICATION

(11) **EP 1 333 871 B1**
(45) Date of publication and mention of the grant of the patent: **31.12.2008**
(21) Application number: 00973237.1
(22) Date of filing: 03.11.2000
(51) Int. Cl.: A61L 33/10, A61L 27/34

(54) **HYDROPHOBIC MULTICOMPONENT HEPARIN CONJUGATES, A PREPARING METHOD AND A USE THEREOF**
HYDROPHOBE MEHRKOMPONENTEN-HEPARINKONJUGATE, EIN VERFAHREN ZUR IHRER HERSTELLUNG UND VERWENDUNG
CONJUGUES D'HEPARINE COMPOSITES HYDROPHOBES, AINSI QUE PROCEDE DE PREPARATION ET UTILISATION DE CEUX-CI

(30) Priority: 24.10.2000 KR 2000062668
(43) Date of publication of application: 13.08.2003
(73) Proprietor: Mediplex Corporation, Korea, Seoul 135-729 (KR)
(72) Inventor: BYUN, Young Ro, Kwangju 506-302 (KR); Moon, Hyun Tae, Kwangju 500-170 (KR)
(74) Representative: Smyth, Gyles Darren
(86) International application number: PCT/KR2000/001255
(87) International publication number: WO 2002/034312

(56) References cited:
- EP-A- 0 947 205
- WO-A-91/16932
- WO-A-99/61481
- DE-A- 4 217 917
- JP-A- 1 262 869
- JP-A- 9 510 736
- JP-A- 11 506 420
- JP-A- 53 057 288
- KR-A- 19990 087 944
- US-A- 4 833 200
- TAE MOON H ET AL: "A novel formulation for controlled release of heparin-DOCA conjugate dispersed as nanoparticles in polyurethane film" BIOMATERIALS, ELSEVIER SCIENCE PUBLISHERS BV., BARKING, GB, vol. 22, no. 3, February 2001 (2001-02), pages 281-289, XP004221086 ISSN: 0142-9612
- LEE YONG-KYU ET AL: "Preparation of slightly hydrophobic heparin derivatives which can be used for solvent casting in polymeric formulation" THROMBOSIS RESEARCH, vol. 92, no. 4, 15 November 1998 (1998-11-15), pages 149-156, XP002279812 ISSN: 0049-3848

## Description

### FIELD OF THE INVENTION

The present invention relates to hydrophobic multicomponent heparin conjugates which are soluble not in water but in specific organic solvents, a preparing method and a use thereof. Particularly, the present invention relates to hydrophobic multicomponent heparin conjugates which are prepared by covalently binding polymer and hydrophobic materials to heparin which is used for treatment of patients as a antithrombogenic agent, a preparing method and a use thereof.

### BACKGROUND

Thromboresistence of vascular walls requires endothelial lining which can be compatible to the platelets and the plasma coagulation system. Unlike the artificial vascular materials, endothelial lining does not induce serine protease activity during coagulation process. However, when coagulability of blood is increased, endothelium inactivates thrombin and other possible coagulation factors. Generally, the inflow site of thrombin is known to be the surface of endothelium cells (B. Awbrey et al. J. Biol. Chem., 254, 4092-4095, 1979), and glycosaminoglycan layer on the endothelial cells is also known to have thrombin-binding sites. It is reported that if those thrombin binding sites are bound to enzymes such as serine protease, the enzymes are inactivated by antithrombin III in blood serum(C. Busch et al., J. Clin. Invest., 69, 726-729, 1982; M. Dryjski et al., Thromb. Res., 32, 355-363, 1983).

After reported that analogous structure for antithrombin binding exists in heparin molecules (J.A. Marcum et al., J. Clin. Invest., 74, 341-350, 1984; J.A. Marcum et al., Biochem. Biophys. Res. Comm., 126, 365-372, 1985), the methods for introducing heparin into or on the surface of solid material to produce endothelium-like thromboresistent properties is already developed and commercialized. Artificial surface, which have obtained endothelium-like thromboresistent properties by using heparin et al. can be widely used for the development of medical techniques.

Among those studies, various methods for improving blood compatibility of macromolecular surface using heparin are developed. Grode et al. synthesized heparin-silicon rubber using cyanuric chloride and radiation grafting (Grode et al., J. Biomed. Mater. Res. Symp., 3, 77-84, 1972). Merrill et al. also synthesized polyvinyl alcohol, which is covalently bound to heparin by glutaraldehyde cross linking (Merrill et al., J. Appl. Physio., 29, 723-728, 1970). Eriksson and Gillerg developed cross-linked heparin monomer in which anionic heparin is absorbed on the surface of polypropylene by heparin cross linking and cationic surfactant (Eriksson and Gillerg, J. Biomed. Mater. Res., 1, 301-312, 1967).

Goosen and Sefton synthesized heparinized styrenebutadiene-styrene elastomer (Goosen and Sefton, Thromb. Res., 20, 543-554, 1980). And, Miyura et al. designed the method of prolongation of plasma recalcification by fixing heparin on sepharose or polyhydroxymetha crylate (Miyura et al., J. Biomed. Mater. Res., 14, 619-630, 1980).

Jacobs et al. synthesized covalent bond complex of heparin and prostaglandin E1 (Jacobs et al., J. Biomed. Mater. Res., 23, 611-630, 1989). The above heparin complex is fixed on urethane by diamino-terminated polyethylene oxides, and this fixed complex is very effective to prohibit thrombin formation. Hennink et al. developed covalent bond complex of heparin and human serum albumin (Hennink et al., Thromb. Res., 29, 1-13, 1983). This complex increases blood compatibility of macromolecular surface, which is in contact with the blood.

Nagaoka et al. reported the results of animal experiments which show that polyethylene oxide (PEO) hydrophilic spacer fixed on macromolecular surface inhibits the attachment of platelets by absorption of blood proteins and dynamic repulsion (Nagaoka et al., Trans. Am. Soc. Artif. Intern. Organs., 10, 76-78, 1987). Inhibition effect of platelet attachment of spacer fixed on heparinized surface was confirmed by Kim and Ebert (Kim and Ebert, Thromb. Res., 26, 43-57, 1982). They proved that anticoagulant activity of fixed heparin increased according to the length of spacer.

And, Tay et al. showed that 1) long spacer such as PEO make binding heparin with antithrombin III and thrombin easier; 2) heparin joined by end groups of polyvinylalcohol is more accessible than heparin joined by amino groups on non-terminal units of heparin macromolecules; 3) heparin units on the surface obstruct the accession of antithrombin III and thrombin (Tay et al., Biomaterials, 10, 11-15, 1989).

Schmer bound heparin covalently to agarose by using spacers such as thiophosgene or carbodiimide (Schmer, Trans. Am. Soc. Artif. Intern. Organs., 18, 321-324, 1972), and this spacer-fixed heparin has advanced binding capacity to the antithrombinIII. Danishefsky and Tzeng produced heparin-agarose macromolecules by using aminoethyl spacer (Danishefsky and Tzeng, Thromb. Res., 4, 237-246, 1974). Park et al. fixed heparin on a biomer by using PEO spacer, and they reported that the length of such spacers should be at least 3,400 Daltons to optimize anticoagulation activity of the fixed heparin (Park et al., J. Biomed. Mater. Res., 22, 977-992, 1988). Also, heparin was fixed on the surface of spacers to optimize anticoagulation activity and to amplify surface density of spacer. (Lin et al., J. Biomed. Mater. Res., 25, 791-795, 1991; Piao et al., Trans. Am. Soc. Artif. Intern. Organs., 38, 638-643, 1992).

And also, spacer systems using heparin was used for synthesis of copolymers. Triblock copolymers such as PEO-PDMS-heparin and PDMS-PEO heparin were synthesized (Grainger et al., J. Biomed. Mater. Res., 22, 231-249, 1988). Above triblock copolymers coated on the segmented polyurethane surface increased nonthrombogenicity even under low flow. Vulic et al. reported the synthetic method of polyurethane-PEO-heparin (Vulic et al., J. Polym. Sci. A, Polym. Chem., 26, 381-391, 1988), which is the modification method of surface by solvent casting method. According to this method, coating is easy but synthesis of heparin complex with polyurethane or polyethylene glycol is not. And, it has some problems of low efficiency and of requiring excessive amount of solvent to suppress cross-linking of heparin during synthesis process.

To overcome the foregoing and other disadvantages, we, the inventors of the present invention, have developed synthetic method of heparin complex comprising heparin and hydrophobic macromolecules in which heparin is combined first with a macromolecule having multiple functional groups and then with hydrophobic materials. The result is a synthesized hydrophobic multicomponent heparin conjugates which are soluble not in water but in organic solvents. And finally, the present invention is accomplished by verifying that this hydrophobic multicomponent heparin conjugates can be coated easily on the surface of all medical instruments comprising macromolecules or metals, and have high anti-thrombosis.

The objective of this invention is to provide hydrophobic multicomponent heparin conjugates which are soluble not in water but in specific organic solvents, a preparing method and a use thereof.

### BRIEF DESCRIPTION OF THE DRAWINGS

FIG. la is a photograph of control glass showing the result of platelet attachment experiment of glass modified with hydrophobic multicomponent heparin conjugates of the present invention.

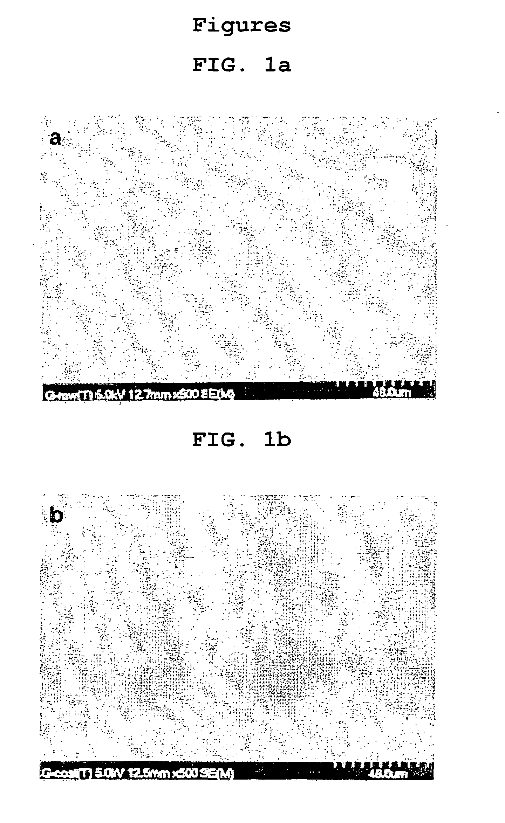

FIG. 1b is a photograph of glass coated with hydrophobic multicomponent heparin conjugates showing the result of platelet attachment experiment of glass modified with hydrophobic multicomponent heparin conjugates of the present invention.

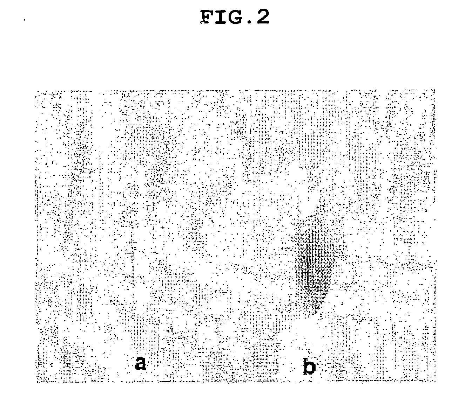

FIG. 2 is a photograph showing the result of blood compatibility experiment of an angiocatheter whose surface was modified with hydrophobic multicomponent heparin conjugates of the present invention.
   (a) A control catheter,
   (b) A catheter coated with hydrophobic multicomponent heparin conjugates.

To accomplish those objectives, the present invention provides a hydrophobic heparin conjugate as defined in Claim 1.

The present invention also provides a method for preparing a hydrophobic heparin conjugate, which method is as defined in Claim 2.

This invention also provides a coating agent which is useful in modifying the surfaces of medical instruments and artificial organs, as defined in Claim 3.

Hereinafter, the present invention is described in detail.

The present invention provides hydrophobic multicomponent heparin conjugates in which heparin is combined with macromolecules and hydrophobic materials.

Hydrophobic multicomponent heparin conjugates of the present invention are the heparin combined with macromolecules containing multiple functional groups, in which binding ratio of heparin and macromolecule can be controlled, and amine group among the functional groups of heparin is used for binding with macromolecules.

Heparin has carboxyl group, hydroxyl group, sulfonic acid group and amine group as functional groups. All the functional groups except amine group exist in the active site of anti-thrombosis; so when those functional groups are used for binding with macromolecules, these heparin-macromolecule conjugates would be in loss of their anti-thrombosis activity.

Synthetic macromolecules, proteins, can be used as macromolecules containing the aforementioned multiple functional groups. For synthetic macromolecule, polyacrylic acid are be used. For proteins, protamine or polylysine are be used.

Hydrophobic multicomponent heparin conjugates of the present invention are produced by combining heparin and macromolecules containing multiple functional groups, and then hydrophobic material is bound thereto. As a result, heparin molecule forms a conjugate by binding with many macromolecules containing multiple functional groups, to which hydrophobic materials are attached. Thus, many functional groups, which are not participated in binding, can be reacted with a hydrophobic material. Finally, hydrophobic multicomponent heparin conjugates of the present invention have hydrophobicity because heparin is combined with hydrophobic macromolecule.

Hydrophobic multicomponent heparin conjugates of the present invention are soluble not in water but in specific organic solvents such as tetrahydrofuran and DMAC (dimethylacetamide). Thus, hydrophobic multicomponent heparin conjugates of the present invention can be easily synthesized by the solvent evaporation method and also they can be easily coated on the surface of macromolecules or metals by the dip coating or spray method. And, for clinical usage, the hydrophobic multicomponent heparin conjugates of the present invention can be used with stability because of their water insolubility.

Heparin in the hydrophobic multicomponent heparin conjugates of the present invention sustains its anti-thrombosis activity even after it is coated with substrates, so it can inhibit blood clotting which might arise on the surface of coated substrates. Therefore, the hydrophobic multicomponent heparin conjugates of the present invention improved the anti-thrombosis effect of the coated surface. On the other hand, heparin contains lots of anions, and thus decreases the friction coefficient of coated surfaces significantly while increasing the water-absorption capacity of surfaces. Thus, the quality of artificial organs and medical instruments such as catheters or insertion tubes, which are in contact with blood can be improved by modification of their surface by coating with the hydrophobic multicomponent heparin conjugates of the present invention, thereby minimizing side-effects caused by thrombus.

The present invention also provides a preparation method of the hydrophobic multicomponent heparin conjugates including steps as follows;
1) Activation step of macromolecules:
2) Binding step of heparin and activated macromolecule:
3) Binding step of hydrophobic materials and functional groups of macromolecules combined with heparin.

Whereas the existing method is to synthesize a triblock copolymer in which heparin is combined with polyurethane and polyethylene glycol, the method of the present invention consists of multistep process including synthesis of graft copolymer in which heparin is combined with macromolecule and hydrophobic material. And according to the present invention, macromolecule itself does not need to have hydrophobicity. The structure of the hydrophobic multicomponent heparin conjugates is different from that of the triblock copolymer. Triblock copolymer has a form in which heparin is combined with the end of PEG-polyurethane chain, but the hydrophobic multicomponent heparin conjugates of the present invention have a form in which macromolecule containing multiple functional groups is combined with heparin itself and then hydrophobic material is combined with functional group of macromolecule.

As a macromolecule in step 1, polyacrylic acid protamine, or polylysine are be used.

To activate above macromolecules, in the preferred embodiment of the present invention, carboxyl group (COOH) of macromolecule is activated by using N,N-dicyclohexylcarbodiimide (DCC), and also 1-ethyl-3-dimethylaminopropyl-carbodiimide hydrochloride (EDC) or 4-p-azidosalicylamido-butylamine (ASBA) can be used.

In step 2, it is preferred that molecular weight of heparin is 1,000-1,000,000 dalton. For the binding heparin with activated macromolecules, a covalent bond that is an amide bond is used.

In step 3, it is preferred that water-solubility of hydrophobic material which is combined with macromolecule is less than 100 mg/mℓ, and has at least one functional group. Octadecylamine (ODA), deoxycholic acid or glycocholic acid are used as a hydrophobic material. Covalent bond by using hydroxyl group, carboxyl group, amine group, thiol group and azide group was used for the binding hydrophobic materials to the functional groups of macromolecules combined with heparin. It is preferred that the above covalent bond is non-degradable bond such as amide bond and urethane bond, or could be degradable bond such as ester bond and anhydride bond.

As mentioned above, hydrophobic multicomponent heparin conjugates of the present invention can be easily synthesized by the solvent evaporation method and also they can be easily coated on the surface of macromolecules or metals by the dip coating or spray method. And, for clinical usage, the hydrophobic multicomponent heparin conjugates of the present invention can be used with stability because of their water insolubility.

Substrates coated with the hydrophobic multicomponent heparin conjugates of the present invention have high anti-thrombosis activity because it can suppress blood clotting, and have a property that decrease friction coefficient of coated surface significantly and increase water-absorption capacity of surface.

Thus, artificial organs and medical instruments such as catheters or insertion tubes which are in contact with blood can be advanced by modification of their surface by coating with the hydrophobic multicomponent heparin conjugates of the present invention, and side-effects caused by thrombus can be minimized by use of the hydrophobic multicomponent heparin conjugates of the present invention.

### EXAMPLES

Practical and presently preferred embodiments of the present invention are illustrative as shown in the following Examples.

However, it will be appreciated that those skilled in the art, on consideration of this disclosure, may make modifications and improvements within the scope of the present invention.

### Example 1: synthesis of heparin, polyacrylic acid, and

### octadecylamine conjugates

### <1-1> Activation of polyacrylic acid

5 g of polyacrylic acid(referred as "PAA" hereinafter) was added to 250 mℓ of N,N-dimethylformamid(referred as "DMF" hereinafter) and dissolved, which thereafter was cooled in an ice bath. To the above mixed solution, 25 mℓ of DMF solution in which 15.5 g of N,N-dicyclohexylcarbodiimide(referred as "DCC" hereinafter) was dissolved, was immediately added. Through 5-minute stirring, DCC had been activated, after which 25 mℓ of DMF solution containing 8.7 g of N-hydroxylsuccinimide(referred as "HOSU" hereinafter) was quickly added to the above mixture. As reaction went by, N,N-dicyclohexylurea (referred as "DCUrea" hereinafter) has been gradually generated and precipitated. After 24 hours of reaction, DCUrea was removed by filtering with 0.2 µm pore size membrane and the mixed solution was just left at 5°C for 12 hours and thereafter, the small amount of remaining DCUrea was filtered and removed completely with the same manner as above. Through the solvent evaporation at room temperature, DMF was removed. Into the solution above, 500 mℓ of methanol was added and stirred for 5 hours, after which DCC and HOSU remaining unreacted were removed by filtering with 0.2 µm membrane. Methanol was removed by vacuum drying at room temperature for 6 hours, and 8.7 g of white powder was finally obtained.

### <1-2> Synthesis of heparin-polyacrylic acid-octadecylamine conjugate (Heparin/PAA/ODA=1:1:20, Molar Ratio)

0.11 g of activated PAA obtained in the above example <1-1> was dissolved into 100 mℓ of DMF, into which 50 mℓ of distilled water containing 0.3 g of heparin was loaded for 5 minutes. Within 30 minutes after reaction began, sediment, a mid-product from combining heparin and activated PAA, was generated and the solution became less clear with it. 5 hours later, 350 mℓ of THF solution containing 0.13 g of octadecylamine(referred as "ODA" hereinafter) was gradually added into the solution for 5 minutes, and was left to react for 5 hours at room temperature. Through the above procedure, white sediment began to be generated and this generation had been completed within 30 minutes after reaction. After the completion of reaction, 400 mℓ of THF and distilled water were removed from the solution by solvent evaporation at room temperature, and the unreacted remaining ODA was removed by dialysis using cellulose ester membrane and acetone as a solvent. Thereafter, acetone was substituted with water and filtered with 0.45 µm filter membrane, resulting in the elimination of unreacted remaining heparin. The white sediment obtained from the above was vacuum dried for 12 hours. As a result, 0.45 g of white powder was obtained.

### <1-3> Synthesis of heparin-polyacrylic acid-octadecylamine conjugate (Heparin/PAA/ODA=1:5:100, Molar Ratio)

0.55 g of activated PAA obtained in the above example <1-1> was dissolved into 100 mℓ of DMF, into which 50 mℓ of distilled water containing 0.3 g of heparin was loaded for 5 minutes. Within 30 minutes after reaction began, sediment, a mid-product from combining heparin and activated PAA, was generated and the solution became less clear with it. 5 hours later, 350 mℓ of THF solution containing 0.67 g of ODA was slowly added into the solution for 5 minutes, and was left to react at room temperature for 5 hours. Through the above procedure, white sediment was generated and the generation was completed within 30 minute. On completion of reaction, 400 mℓ of THF and distilled water were removed from the above solution by solvent evaporation at room temperature and unreacted ODA was eliminated by dialysis using a cellulose ester membrane and acetone as a solvent. Thereafter, acetone solvent was replaced with water and filtered with 0.45 µm membrane resulting in removing unreacted heparin. The white sediment obtained from that procedure was vacuum dried for 12 hours, from which 1.1 g of white powder was obtained.

### <1-4> Synthesis of heparin-polyacrylic acid-octadecylamine conjugate (Heparin/PAA/ODA=1:10:200, Molar Ratio)

1.11 g of activated PAA obtained in the above example <1-1> was dissolved into 100 mℓ of DMF, into which 50 mℓ of distilled water containing 0.3 g of heparin was loaded for 5 minutes. Within 30 minutes after reaction began, sediment, a mid-product from combining heparin and activated PAA was generated and the solution began to be less clear. 5 hours later, 350 mℓ of THF solution in which 1.35 g of ODA was dissolved was gradually added into the solution for 5 minutes, after which it was left to react at room temperature for 5 hours. Finally, white sediment was generated and the generation was completed within 30 minutes. As the reaction was completed, 400 mℓ of THF and distilled water were eliminated from the mixed solution by solvent evaporation at room temperature and unreacted ODA was also removed by dialysis using a cellulose ester membrane and acetone as a solvent. Later on, acetone solvent was substituted with water and unreacted heparin was removed through filtering with 0.45 µm membrane. The white sediment obtained from that procedure was vacuum dried for 12 hours, from which 2.0 g of white powder was obtained.

### Example 2: synthesis of heparin, protamine and

### deoxycholic acid conjugates

### <2-1> Activation of deoxycholic acid

11.8 g of deoxycholic acid(referred as "DOCA" hereinafter) was dissolved into 100 mℓ of DMF and cooled in an ice bath thereafter. To that mixed solution, 25 mℓ of DMF solution in which 7.4 g of DOC was dissolved was quickly added. On stirring for 5 minutes, DCC became activated and 25 mℓ of DMF solution in which 4.14 g of HOSU was dissolved was rapidly added thereto. As reaction went by, DCUrea was gradually precipitated. After 24 hours of reaction, DCUrea was removed by filtering with 0.2 µm membrane and the solution was suspended at 5°C for 12 hours, after which the small amount of remaining DCUrea was eliminated completely by filtering as the same manner above. 100 mℓ of acetonitrile was added to the reactive solution in which DMF was removed through solvent eavaporation at room temperature resulting in precipitation of white solid body. That white solid body was obtained through filtering using 0.2 µm membrane followed by vacuum drying at room temperature for 5 hours, from which 12.5 g of activated DOCA was obtained.

### <2-2> Synthesis of hydrophobic protamine (protamine-DOCA)

0.5 g of protamine was dissolved into 50 mℓ of distilled water, into which 50 mℓ of DMF solution containing 0.27 g of activated DOCA obtained in the above example <2-1> was gradually added. The solution was suspended at room temperature for 5 hours while coupling reaction was under going. DMF and distilled water were removed from the above mixed solution through solvent evaporation at room temperature, after which the solution was washed with 100 mℓ of distilled water three times resulting in the elimination of unreacted protamine and obtaining of white solid sediment. The solid sediment was washed with 100 mℓ of methanol. After removing unreacted activated DOCA by filtering with 0.45 µm membrane, white powder was obtained through vacuum drying. The unreacted remaining amino acid residue of protamine was eliminated using 1 hour blocking reaction in 100 mℓ, 1.0 wt% acetaldehyde solution and in 100 mℓ, 50% methanol solution. After the reaction was finished, unreacted acetaldehyde was removed by washing with distilled water three times and vacuum dried, from which 0.55 g of hydrophobic protamine (protamine-DOCA) in which DOCA was partly coupled with protamine was obtained.

### <2-3> Esterification of protamine-DOCA

0.5 g of activated protamine-DOCA obtained in the above example <2-2> was dissolved in 80 mℓ of DMF and this solution was added into 10 mℓ of DMF solution containing 1.5 g of DCC without hesitation. Upon 5 minute stirring, DCC was activated and 10 mℓ of DMF containing 0.84 g of HOSU was added thereto. As reaction went by, the small amount of DCUrea was precipitated. After 24 hours of reaction at room temperature, DCUrea was removed through filtering using 0.2 µm membrane and DMF was also eliminated from the above solution by solvent evaporation at room temperature. The white powder obtained therefrom was washed with 100 mℓ methanol twice and filtered with 0.2 µm membrane, so that white solid body was obtained. The solid body was vacuum dried at room temperature for 5 hours. As a result, 0.45 g of hydrophobic protamine (protamine-DOCA) activated by esterification was obtained.

### <2-4> Synthesis of heparin-protamine-DOCA conjugate

0.5 g of activated protamine-DOCA obtained in the above example <2-3> was dissolved in 100 mℓ of DMF solution, into which 50 mℓ of distilled water containing 0.92 g of heparin was loaded. Within 30 minutes after reaction began, sediment combined by condensation reaction was precipitated, and the generation of the sediment was completed in 30 minutes. When the reaction has continued for 5 hours, distilled water and DMF were removed from the reaction solution by solvent evaporation at room temperature, from which white powder was obtained. The unreacted active protamine-DOCA was removed by dialysis using cellulose ester membrane and methanol as a solvent. Methanol was substituted with water and filtered using 0.45 µm membrane, resulting in elimination of unreacted heparin. White sediment obtained therefrom was vacuum dried at room temperature for 12 hours and finally 1.05 g of white powder was obtained.

### Example 3: synthesis of heparin, protamine and

### glycocholic acid conjugate

### <3-1> Activation of glycocholic acid

9.3 g of glycocholic acid (referred as "GOCA" hereinafter) was dissolved in 100 mℓ of DMF, into which 25 mℓ of DMF solution containing 6.6 g of DCC was added. Through 5 minute stirring, DCC was activated. 25 mℓ of DMF solution in which 3.7 g of HOSU was dissolved was added thereto. While esterification was going under, DCUrea began to appear and precipitate slowly. After 24 hours of reaction at room temperature, DCUrea was removed by filtering with the 0.2 µm membrane and the solution was suspended further at 5°C for 12 hours in order for the rest of DCUrea to be precipitated. The rest amount of precipitated DCUrea was completely removed with the same manner as explained above. The concentration of the solution was fixed at 15 mℓ at room temperature, and 100 mℓ of acetonitrile was added thereto, so as to precipitate white solid. By filtering the solid with 0.2 µm membrane, white powder was obtained and continued to be vacuum dried for 5 hours. Finally, 7.0 g of activated GOCA was obtained.

### <3-2> Synthesis of hydrophobic protamine (protamine-GOCA)

0.5 g of protamine was dissolved in 50 mℓ of distilled water, into which 50 mℓ of DMF solution containing 0.31 g of GOCA obtained in the above example <3-1> was added slowly. After 5 hours of coupling reaction at room temperature, the mixed solution was solvent evaporated in order to remove distilled water and DMF. The solution was, then, washed with 100 mℓ of distilled water three times. As a result, unreacted protamine was removed and white solid sediment was obtained. The obtained sediment was washed with 100 mℓ of methanol and filtered with 0.45 µm membrane, so as to remove unreacted active GOCA. Through the vacuum dry thereof, white powder was obtained. The above white powder was loaded into 100 mℓ, 50% methanol solution containing 1.0 wt% acetaldehyde for 1 hour, during which blocking reaction was under going. The remaining unreacted amino acid residue in protamine was eventually removed therefrom. After the reaction was completed, the solution was washed with distilled water three times, and vacuum dried. Finally, 0.6 g of hydrophobic protamine-GOCA in which GOCA was partly combined to protamine was obtained.

### <3-3> Esterification of protamine-GOCA

0.5 g of protamine-GOCA obtained in the above example <3-2> was dissolved in 80 mℓ of DMF, and 10 mℓ of DMF solution containing 1.5 g of DCC was added quickly thereto. During 5 minutes of stirring, DCC was activated, and further 10 mℓ of DMF solution in which 0.84 g of HOSU was dissolved was added. As reaction was going on, the small amount of DCUrea was precipitated. After 24 hours of reaction at room temperature, the solution was filtered with 0.2 µm membrane resulted in removing DCUrea, and solvent evaporated at room temperature resulted in removing DMF. White powder obtained therefrom was washed with 100 mℓ of methanol twice and filtered with 0.2 µm membrane, from which white solid sediment was obtained. Through 5 hours vacuum drying at room temperature thereof, 0.4 g of hydrophobic protamine-GOCA activated by esterification was obtained.

### <3-4> Synthesis of heparin-protamine-GOCA conjugate

0.5 g of activated protamine-GOCA obtained in the above example <3-3> was dissolved in 100 mℓ of DMF, and 50 mℓ of distilled water in which 0.78 g heparin was dissolved was mixed thereto for 5 minutes. Within 30 minutes after reaction began, sediment was generated by condensation reaction, and the generation was completed in 30 minutes. After 5 hours of reaction at room temperature, the above solution was solvent evaporated in order to remove distilled water and DMF. The unreacted active protamine-GOCA was removed from the white powder obtained therefrom by dialysis using cellulose ester membrane and methanol as a solvent. Methanol was substituted with water and the solution was filtered with 0.45 µm membrane, so that the unreacted heparin was removed and white sediment was obtained. The sediment was vacuum dried at room temperature for 12 hours and finally 0.9 g of white powder was obtained.

### Example 4: Synthesis of heparin, polylysine and DOCA

### conjugate

### <4-1> Synthesis of polylysine and DOCA conjugate

1.0 g of polylysine(referred as "PLL" hereinafter) was dissolved in 50 mℓ of distilled water, and 50 mℓ of DMF solution in which 0.98 g of activated DOCA was dissolved was slowly added thereto. After 5 hours of coupling reaction, the solution was solvent evaporated at room temperature, so that distilled water and DMF were removed. The solution was washed with 100 mℓ of distilled water three times in order to remove unreacted PLL resulting in the generation of white solid sediment. The solid sediment was washed with 100 mℓ of methanol and filtered with 0.45 µm membrane in order to remove unreacted active DOCA. This sediment was further vacuum dried leading to obtaining white powder. The white powder was loaded into 100 mℓ, 50% methanol solution containing 1.0 wt% acetaldehyde for 1 hour, during which blocking reaction was under going. As a result, the remaining unreacted amino acid residue of PLL was removed. After the reaction was completed, unreacted acetaldehyde was removed by washing three times with distilled water, and further by vacuum drying, 1.5 g of hydrophobic PLL-DOCA in which DOCA was partly combined with PLL was obtained.

### <4-2> Esterification of PLL-DOCA

1.0 g of PLL-DOCA obtained in the above example <4-1> was dissolved in 80 mℓ of DMF, and then, 10 mℓ of DMF solution containing 2.0 g of DCC was slowly added thereto. Through 5 minutes stirring, DCC began to be activated, and 10 mℓ of DMF solution containing 1.12 g HOSU was added again. As reaction went on, a small amount of DCUrea was generated. 24 hours after the reaction at room temperature, the solution was filtered with 0.2 µm membrane in order to remove DCUrea, and solvent evaporated at room temperature in order to remove DMF. The white powder obtained therefrom was washed twice with 100 mℓ of methanol and filtered with 0.2 µm membrane, so that white solid sediment was obtained. The sediment was vacuum dried for 5 hours at room temperature. As a result, 0.9 g of PLL-DOCA activated by esterification was obtained.

### <4-3> Synthesis of heparin-PLL-DOCA conjugate

0.7 g of activated PLL-DOCA obtained in the above example <4-2> was dissolved in 100 mℓ of DMF, and 50 mℓ of distilled water in which 0.95 g of heparin was dissolved was loaded thereto for 5 minutes. Within 30 minutes after reaction began, sediment combined by condensation reaction began to be generated, and the generation was completed in 30 minutes. After 5 hours of reaction at room temperature, the solution was solvent evaporated to remove distilled water and DMF, from which white powder was obtained. The unreacted active PLL-DOCA was eliminated by dialysis using cellulose ester membrane and methanol as a solvent. Then, methanol was substituted with water and the solution was filtered with 0.45 µm membrane in order to remove unreacted heparin, from which white sediment was obtained. The sediment was vacuum dried at room temperature for 12 hours, and finally 1.1 g of white powder was obtained.

### Example 5: Synthesis of heparin, PLL and GOCA conjugate

### <5-1> Synthesis of PLL and DOCA conjugate (PLL-GOCA)

1.0 g of PLL was dissolved in 50 mℓ of distilled water, and 50 mℓ of DMF solution, in which 0.98 g of activated GOCA obtained in the above example <3-1> was dissolved, was added slowly thereto. After 5 hours of coupling reaction at room temperature, the solution was solvent evaporated at room temperature to remove distilled water and DMF. And then, the rest of the solution was washed with 100 mℓ of distilled water three times resulting in removing of unreacted PLL, from which white solid sediment was obtained. The above solid sediment was washed with 100 mℓ of methanol, filtered with 0.45 µm membrane to remove unreacted active GOCA, and further, vacuum dried. As a result, white powder was obtained. The unreacted amino acid residue remaining in PLL was removed through 1 hour blocking reaction in 100 mℓ, 50% methanol solution containing 1.0 wt% acetaldehyde. After the reaction was completed, the solution was washed with distilled water three times, so that the unreacted acetaldehyde was removed. By vacuum drying thereof, 1.5 g of hydrophobic PLL-GOCA in which GOCA was partly combined to PLL was obtained.

### <5-2> Esterification of PLL-GOCA

1.5 g of PLL-GOCA obtained in the above example <5-1> was dissolved in 100 mℓ of DMF, and then 10 mℓ of DMF solution in which 1.45 g of DCC was dissolved was added quickly. During 5 minute stirring, DCC was activated. Again, 10 mℓ of DMF solution in which 1.67 g of HOSU was dissolved was added thereto. As reaction was going on, a small amount of DCUrea was generated. 24 hours after the reaction at room temperature, the solution was filtered with 0.2 µm membrane to remove DCUrea and solvent evaporated at room temperature to remove DMF. The white powder obtained therefrom was washed with 100 mℓ of methanol twice, and filtered with 0.2 µm membrane resulting in obtaining white solid. The white solid was, then, vacuum dried at room temperature for 5 hours and finally 1.3 g of PLL-GOCA activated by esterification was obtained.

### <5-3> Synthesis of heparin-PLL-GOCA conjugate

1.0 g of activated PLL-GOCA obtained in the above example <5-2> was dissolved in 100 mℓ of DMF, and then 50 mℓ of distilled water containing 0.95 g of heparin was loaded for 5 minutes. Within 30 minutes after reaction began, sediment combined by condensation reaction began to be generated and the solution became less clear. That generation was completed in 30 minutes. After 5 hours of reaction, the solution was solvent evaporated at room temperature resulting in removing distilled water and DMF, from which white powder was obtained. The unreacted active PLL-GOCA was removed from the white powder by dialysis using cellulose ester membrane and methanol as a solvent. Then, methanol was substituted with water and the unreacted heparin was removed by filtering the solution with 0.45 µm membrane. The white sediment obtained therefrom was vacuum dried at room temperature for 12 hours, from which 1.6 g of white powder was obtained.

### Example 6: Analysis of hydrophobic multicomponent

### heparin conjugates

### <6-1> Structure analysis using FT-IR spectrum

In order to analyze the structures of the hydrophobic multicomponent heparin conjugates synthesized according to the above manner, FT-IR spectrum (Infrared spectrum, Perkin Elmer Ltd.) was inspected. As a result, in case of heparin/PAA/ODA conjugate synthesized in example <1-3>, C=O double bond, an amide bond, was remarkable in the range of 170 cm-1 of infrared spectrum, and so was hydroxyl group of heparin in the range of 3500 cm-1, and methyl group of ODA showed its remarkable wave length in the range of 2800 - 2900 cm-1.

### <6-2> Coating of hydrophobic multicomponent heparin conjugates

1.0 mℓ of co-solvent(THF:N,N-dimethylacetamide, 1:1 v/v%) was added to the 25 mg of hydrophobic multicomponent heparin conjugates synthesized in the example <1-3> until the total concentration reached to 2.5 wt%, after which the solution was treated with ultrasonic wave at 40°C for 1 hour resulting in preparation of clear solution. The surfaces of an angiocatheter(2.5 cm in diameter, 5 cm in length) made by polyurethane at 25°C and a glass(0.5 cm x 0.5 cm) were coated once each with the above solution using dipping coating method. The catheter and the glass coated with hydrophobic multicomponent heparin conjugates were dried at room temperature for 30 minutes, followed by 5 hours vacuum drying at room temperature, resulting in complete removal of remaining solvent. Though the coated surfaces of the glass or the catheter were a little unclear, no precipitation phenomenon was observed.

### <6-3> Peeling test of coated multicomponent heparin conjugates in aqueous solution

The glass and the catheter each coated with hydrophobic multicomponent heparin conjugates from the above example <1-3> with the same method as the above example <6-2> were put in a falcon tube filled with 10 mℓ PBS (pH=7.5, I=0.15) and shaken at 37°C, 100 rpm, for 12 hours. When the reaction completed, azure A solution(10 mg/mℓ water) was added thereto, and dye reaction was accomplished(H.J. Conn, "Biological Stains", The Williams and Wilkins., MD, p96-105, 1961). As a result, no color change in the dye solution was detected. Therefore, it is confirmed that if heparin/PAA/ODA, hydrophobic multicomponent heparin conjugates of the present invention is used as coating material, they have great adhesiveness to the materials like a glass or other high polymers but have no peeling phenomenon by swelling in aqueous solutions.

### <6-4> Azure A experiment

A glass or a catheter which had been tested for peeling in the above example <6-3> was immersed in 5 mℓ azure A solution (10 mg azure A/mℓ water), after which the solution was suspended at room temperature for 5 hours. As time went by, the surface of a material coated with hydrophobic multicomponent heparin conjugates showed color change and 5 hours later the dye solution turned into deep purple. Meanwhile, other materials, which were not coated with hydrophobic multicomponent heparin conjugates showed no color change. With those facts, it became certain that hydrophobic multicomponent heparin conjugates have been adhered to the surface of a material with stability while maintaining the unique characteristics of heparin, and anti-thrombus activity.

### <6-5> Contact angle analysis

The catheter(5 cm) coated with hydrophobic multicomponent heparin conjugates(2.5 wt%) with the same manner as the above example <6-2> and the catheter without coating were immersed in 10 mℓ distilled water, and left at room temperature for 5 minutes. As a result, water spreading was observed with the catheter coated with the hydrophobic multicomponent heparin conjugates of the present invention. On the other hand, water spreading of the catheter without coating was decreased whereas its water contacting angle was increased.

### <6-6> Platelet adherence experiment

For the platelet adhesion test, platelet-poor-plasma (referred as "PPP" hereinafter) was separated from a rabbit. More particularly, a New Zealand rabbit (Sunrise Scientific Co.) weighing 2.5 to 3.0 kg, was anaesthetized with ethyleter, and then, 36 mℓ of blood was pulled out with a 50 mℓ syringe using cardiac puncher method. 4 mℓ of 3.8% sodium citrate had been put in that syringe in advance in order to prohibit blood clotting. The blood was centrifuged at 4°C with 1440 rpm for 10 minutes, from which a fresh rabbit PPP was obtained. The platelet concentration in plasma was measured with Coulter counter(Coulter electronics Ltd, UK) and the preferable concentration of platelet was 4.2 x 10⁵ cells/µℓ. The separated PPP was used for the test within 3 hours after preparation.

The glass (0.5 x 0.5 cm) coated with hydrophobic multicomponent heparin conjugates of the present invention(2.5 wt%) and the control glass without coating were put into tubes each having 1 ml of separated fresh rabbit PPP, and suspended in 37°C stirring water bath for 1 hour. 1 hour later, PPP was removed from each tube and the glasses were washed with PBS three times for complete removing of PPP. Each of the above glasses were treated with 3 mℓ of 2.5% glutaraldehyde for platelet fixing for 4 hours, after which they were washed with EtOH aqueous solution according to the following steps. Step 1; 5 mℓ 50% EtOH for 1 hr, step 2; 5 mℓ 80% EtOH for 1 hr, and step 3; 5 mℓ 100% EtOH for 1 hr. Each glass from which ethyl alcohol was gotten rid of was treated with liquid nitrogen, and thereafter freeze-dried for 6 hours. Each glass was observed with SEM to check the platelet adhesion. As a result, it was confirmed that blood compatibility of the glass coated with hydrophobic multicomponent heparin conjugates of the present invention was largely improved, compared to the control glass.

### <6-7> Blood clotting experiment

For the blood-clotting test, whole blood was separated from a rat. Particularly, a Sprague-Dawley rat weighing 250-300 g(Korea Biolink) was anaesthetized with ethyleter, and then, 3.6 mℓ blood was taken with 50 mℓ syringe using cardiac puncher method. 0.4 mℓ of 3.8% sodium citrate had been put in that syringe in advance to prohibit blood clotting. The blood was used for the test immediately after being taken.

The blood vessel catheter(1 cm long) coated with 25 mg of hydrophobic multicomponent heparin conjugates(2.5 wt%) synthesized according to the above example <3-1> and the other catheter without coating were immersed in whole blood of a rat prepared as mentioned above for 1 hour and then, washed with 1^{st} distilled water softly and photographed immediately thereafter. The result was that the catheter coated with hydrophobic muticomponent heparin conjugates of the present invention showed no aggregation of blood components at all, while the control catheter without coating had strong aggregation of blood components in 1 hour after the immersion.

### INDUSTRIAL APPLICABILITY

As shown above, the hydrophobic multicomponent heparin conjugates of the present invention are only soluble in specific organic solvents, and they can be easily coated to macromolecules or the surface of metal by the methods of solvent evaporation, dip coating or spray. Meanwhile, the conjugates are not soluble in water, so that they can be stably used in clinical treatment after coating. Furthermore, the hydrophobic multicomponent heparin conjugates of the present invention keep their original characteristics of anti-thrombus activity after being coated to the substrate. More precisely, the conjugates enhance the anti-thrombus activity of the coated surfaces by restraining blood clotting of the coated surfaces. And also, the hydrophobic multicomponent heparin conjugates of the present invention induce that coated substrates become wet well by decreasing friction coefficient of the coated surfaces, and increasing water-absorption capacity of the coated surfaces. Thus, the hydrophobic multicomponent heparin conjugates of the present invention can be very useful as coating agents restraining the side-effects of thrombus and modifying the surfaces of artificial organs and medical devices contacting blood, for example, catheter, insertion tube, etc.

Those skilled in the art will appreciate that the conceptions and specific embodiments disclosed in the foregoing description may be readily utilized as a basis for modifying or designing other embodiments for carrying out the same purposes of the present invention. Those skilled in the art will also appreciate that such equivalent embodiments do not depart from the scope of the invention as set forth in the appended claims.

## Claims

1. A hydrophobic heparin conjugate composed of heparin, macromolecules containing multiple functional groups, and hydrophobic materials, wherein said conjugate, represented as heparin-macromolecules-hydrophobic materials, is selected from
(1) heparin-polyacrylic acid-octadecylamine,
(2) heparin-protamine-deoxycholic acid,
(3) heparin-protamine-glycocholic acid,
(4) heparin-polylysine-deoxycholic acid, and
(5) heparin-polylysine-glycocholic acid,
wherein the macromolecules containing multiple functional groups are conjugated to the heparin by amide bonds at amine groups among the functional groups of the heparin, and the hydrophobic materials are conjugated to functional groups of the macromolecules by covalent bonds.

2. A method for preparing a hydrophobic heparin conjugate as defined in claim 1, comprising the following steps: 1) bonding macromolecules containing multiple functional groups to heparin by amide bonds at amine groups among the functional groups of the heparin; 2) bonding hydrophobic materials to functional groups of the macromolecules by covalent bonds.

3. A coating agent which is useful in modifying the surfaces of artificial organs and medical instruments, comprising hydrophobic heparin conjugates as set forth in claim 1.

## Patentansprüche

1. Hydrophobes Heparinkonjugat, bestehend aus Heparin, multiple funktionelle Gruppen enthaltenden Makromolekülen, und hydrophoben Materialien, wobei das Konjugat, das als Heparin-Makromoleküle-hydrophobe Materialien dargestellt ist, ausgewählt ist aus
(1) Heparin-Polyacrylsäure-Octadecylamin,
(2) Heparin-Protamin-Desoxycholsäure,
(3) Heparin-Protamin-Glycocholsäure,
(4) Heparin-Polylysin-Desoxycholsäure, und
(5) Heparin-Polylysin-Glycocholsäure,
wobei die multiple funktionelle Gruppen enthaltenden Makromoleküle bei Amingruppen durch Amidgruppen unter den funktionellen Gruppen des Heparins mit dem Heparin konjugiert werden, und die hydrophoben Materialien durch kovalente Bindungen mit funktionellen Gruppen der Makromoleküle konjugiert werden.

2. Verfahren zum Herstellen eines hydrophoben Heparinkonjugats wie in Anspruch 1 definiert, umfassend die folgenden Schritte: 1) Binden der multiple funktionelle Gruppen enthaltenden Makromoleküle an Heparin durch Amidgruppen bei Amingruppen unter den funktionellen Gruppen des Heparins; 2) Binden der hydrophoben Materialien an funktionelle Gruppen der Makromoleküle durch kovelente Bindungen.

3. Beschichtungsmittel, das beim Modifizieren von Oberflächen von künstlichen Organen und medizinischen Instrumenten nützlich ist, umfassend hydrophobe Heparinkonjugate nach Anspruch 1.

## Revendications

1. Conjugué d'héparine hydrophobe composé d'héparine, des macromolécules contenant de multiples groupes fonctionnels, et des matériaux hydrophobes, dans lequel ledit conjugué, représenté en tant que héparine-macromolécules-matériaux hydrophobes, est sélectionné parmi
(1) héparine-acide polyacrylique-octadécylamine,
(2) héparine-protamine-acide déoxycholique,
(3) héparine-protamine-acide glycocholique,
(4) héparine-polylysine-acide déoxycholique, et
(5) héparine-polylysine-acide glycocholique,
dans lequel les macromolécules contenant les multiples groupes fonctionnels sont conjuguées à l'héparine par des liaisons amides au niveau de groupes amines parmi les groupes fonctionnels de l'héparine, et les matériaux hydrophobes sont conjugués aux groupes fonctionnels des macromolécules par des liaisons covalentes.

2. Procédé pour préparer un conjugué d'héparine hydrophobe tel que défini dans la revendication 1, comprenant les étapes suivantes: 1) lier les macromolécules contenant de multiples groupes fonctionnels à l'héparine par des liaisons amides au niveau de groupes amines parmi les groupes fonctionnels de l'héparine; 2) lier les matériaux hydrophobes à des groupes fonctionnels des macromolécules par des liaisons covalentes.

3. Agent de revêtement utile pour modifier les surfaces des organes artificiels et des instruments médicaux, comprenant des conjugués d'héparine hydrophobe tels que décrits dans la revendication 1.
